(19) **Europäisches Patentamt**

**European Patent Office**

**Office européen des brevets**

(11) **EP 1 139 114 A2**

(12) **EUROPEAN PATENT APPLICATION**

(43) Date of publication:
04.10.2001 Bulletin 2001/40

(51) Int Cl.$^7$: **G01R 33/567**

(21) Application number: **01302791.7**

(22) Date of filing: **26.03.2001**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **27.03.2000 US 536013**

(71) Applicant: **GE Medical Systems Global Technology Company LLC Waukesha, Wisconsin 53188 (US)**

(72) Inventors:
• **Maier, Cynthia Faye Fort Lee, New Jersey 07024 (US)**
• **Tan, Guosheng Waukesha, Wisconsin 53186 (US)**

(74) Representative: **Goode, Ian Roy GE LONDON PATENT OPERATION, Essex House, 12/13 Essex Street London WC2R 3AA (GB)**

(54) **Slice ordering method for breath-hold abdominal MR imaging**

(57) A fast spin echo pulse sequence (Fig 3) is employed to acquire MR image data for a set of abdominal slice images (10). The slices are grouped into slabs (12, 14) and each slab is acquired during a patient breath-hold. A navigator pulse sequence may be performed after initiation of each breath-hold to acquire navigator data that enables the slabs (12, 14) to be registered retrospectively, or enables a scan parameter to be adjusted prospectively (256) to acquire each slab (12, 14) in registration.

FIG. 4

**Description**

The field of the invention is nuclear magnetic resonance imaging ("MRI") methods and systems. More particularly, the invention relates to the acquisition of MRI data for a set of slice images through an abdominal organ.

When a substance such as human tissue is subjected to a uniform magnetic field (polarizing field $B_0$), the individual magnetic moments of the spins in the tissue attempt to align with this polarizing field, but precess about it in random order at their characteristic Larmor frequency. If the substance, or tissue, is subjected to a magnetic field (excitation field $B_1$) which is in the x-y plane and which is near the Larmor frequency, the net aligned moment, $M_z$, may be rotated, or "tipped", into the x-y plane to produce a net transverse magnetic moment $M_t$. A signal is emitted by the excited spins after the excitation signal $B_1$ is terminated, this signal may be received and processed to form an image.

When utilizing these signals to produce images, magnetic field gradients ($G_x$ $G_y$ and $G_z$) are employed. Typically, the region to be imaged is scanned by a sequence of measurement cycles in which these gradients vary according to the particular localization method being used. The resulting set of received NMR signals are digitized and processed to reconstruct the image using one of many well known- reconstruction techniques.

Object motion during the acquisition of NMR image data produces both blurring and "ghosts" in the phase-encoded direction. Ghosts are particularly apparent when the motion is periodic, or nearly so. For most physiological motion each view of the NMR signal is acquired in a period short enough that the object may be considered stationary during the acquisition window. In such case the blurring and ghosting is due to the inconsistent appearance of the object from view to view. Motion that changes the appearance between views such as that produced by a patient moving, by the respiration or the cardiac cycle, or by peristalsis, is referred to hereinafter as "view-to-view motion". Motion may also change the amplitude and phase of the NMR signal as it evolves during the pulse sequence and such motion is referred to hereinafter as "in-view motion".

Both blurring and ghosting can be reduced if the data acquisition is synchronized with the functional cycle of the object to reduce view-to-view motion. This method is known as gated NMR scanning, and its objective is to acquire NMR data at the same point during successive functional cycles so that the object "looks" the same in each view. The drawback of gating is that NMR data may be acquired only during a small fraction of the object's functional cycle, and even when the shortest acceptable pulse sequence is employed, the gating technique can significantly lengthen the data acquisition.

Another proposed method for eliminating ghost artifacts is disclosed in U.S. Patent No. 4,567,893, issued on February 4, 1986. This prior patent teaches that the distance in the image between the ghosts and the object being imaged is maximized when the NMR pulse sequence repetition time is an odd multiple of one-fourth of the duration of the periodic signal variation. This can be used to alleviate ghosts due to respiratory motion. While this method, indeed, improves image quality, it does impose a constraint on the NMR pulse sequence repetition time and it often results in a longer total scan time. It also assumes that the motion is periodic.

Yet another method for reducing the undesirable effects due to periodic signal variations is disclosed in U.S. Patent No. 4,706,026 issued on November 10, 1987 and entitled "A Method For Reducing Image Artifacts Due To Periodic Variations In NMR Imaging." In one embodiment of this method, an assumption is made about the signal variation period (e.g. due, for example, to patient respiration) and the view order is altered from the usual monotonically increasing phase-encoding gradient to a preselected order.

For a given signal variation period, a view order is chosen so as to make the NMR signal variation as a function of the phase-encoding amplitude be at a desired frequency. In one embodiment, the view order is selected such that the variation period appears to be equal to the total NMR scan time (low frequency) so that the ghost artifacts are brought as close to the object being imaged as possible. In another embodiment (high frequency), the view order is chosen to make the variation period appear to be as short as possible so as to push the ghost artifacts as far from the object as possible.

This prior method is effective in reducing artifacts, and is in some respects ideal if the variation is rather regular and at a known frequency. On the other hand, the method is not very robust if the assumption made about the motion temporal period does not hold (e.g., because the patient's breathing pattern changes or is irregular). If this occurs, the method loses some of its effectiveness because the focusing of the ghosts, either as close to the object or as far from the object as possible, becomes blurred. A solution to this problem is disclosed in U.S. Patent No. 4,663,591 which is entitled "A Method For Reducing Image Artifacts Due To Periodic Signal Variations in NMR Imaging." In this method, the non-monotonic view order is determined as the scan is executed and is responsive to changes in the period so as to produce a desired relationship (low frequency or high frequency) between the signal variations and the gradient parameter. The effectiveness of this method, of course, depends upon the accuracy of the means used to sense the patient motion, and particularly, any variations in the periodicity of that motion.

The most successful method for correcting MR images for motion artifacts employs navigator signals acquired during the scan. As described in U.S. Patent No. 4,937,526, such navigator signals are acquired periodically during the scan, and the information in these signals may be used to correct the image data for patient motion.

Respiratory motion is the most difficult problem when performing abdominal MR imaging. One solution is to acquire all the image data using motion insensitive techniques such as a single shot, fast spin echo ("SSFSE") or a single-shot echo planar ("EPI") sequence which acquire data for an entire image in one breath-hold. However, neither of these techniques is optimized for obtaining $T_2$-weighted abdominal images. images acquired with SSFSE have more blurring and may have too much $T_1$ contrast due to the stimulated echo contribution to the acquired signals. Single-shot EPI, on the other hand, is not reliable enough for routine clinical imaging of the abdominal due to magnetic susceptibility gradients caused by the air in the lungs and digestive tract. In addition, neither of these techniques can be sued to produce $T_1$-weighted images with acceptable contrast for abdominal imaging

The present invention is a method for acquiring a set of slice images from the abdominal region of a patient without producing motion artifacts caused by patient respiration. More particularly, the set of slices is divided into a plurality of successive slabs, in which each slab is comprised of a plurality of slices that can be acquired in a single breath-hold using the prescribed pulse sequence. Each slab is then acquired by initiating a patient breath-hold and acquiring MR image data for all the slices in the slab using the selected pulse sequence. Images are reconstructed from the acquired MR image data.

Another aspect of the invention is to measure the patient position prior to each slab acquisition and alter a scan parameter on the MRI system such that successive slabs are acquired in a registered manner. A navigator pulse sequence is used to measure patient position prior to each slab acquisition, and slab location is adjusted to account for patient movement away from the reference position during the scan.

Embodiments of the invention will now be described, by way of example, with reference to the accompanying drawings, in which:-

Fig. 1 is a block diagram of an MRI system which employs the present invention;

Fig. 2 is an electrical block diagram of the transceiver which forms part of the MRI system of Fig. 1;

Fig. 3 is a graphic representation of a fast spin echo (FSE) pulse sequence used to practice the preferred embodiment of the invention;

Fig. 4 is a pictorial representation of a patient illustrating the order in which slice image data is acquired using the pulse sequence of Fig. 3;

Fig. 5 is a flow chart of the steps performed by the MRI system of Fig. 1 to acquire the image data;

Fig. 6 is a flow chart of the steps performed by the MRI system of Fig. 1 to acquire image data according to an alternative embodiment.

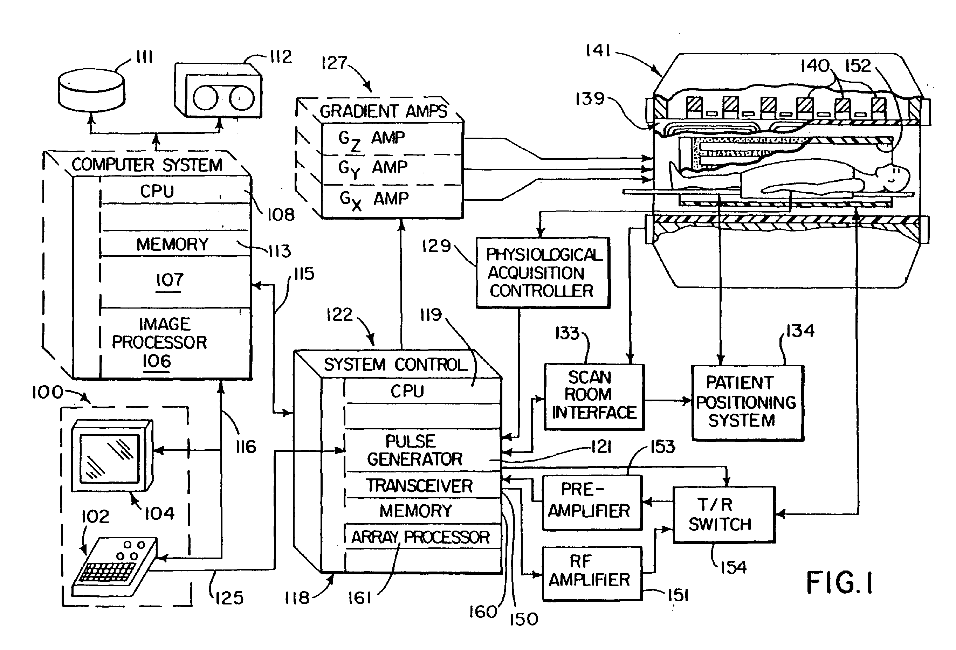

Referring first to Fig. 1, there is shown the major components of a preferred MRI system which incorporates the present invention. The operation of the system is controlled from an operator console 100 which includes a keyboard and control panel 102 and a display 104. The console 100 communicates through a link 116 with a separate computer system 107 that enables an operator to control the production and display of images on the screen 104. The computer system 107 includes a number of modules which communicate with each other through a backplane. These include an image processor module 106, a CPU module 108 and a memory module 113, known in the art as a frame buffer for storing image data arrays. The computer system 107 is linked to a disk storage 111 and a tape drive 112 for storage of image data and programs, and it communicates with a separate system control 122 through a high speed serial link 115.

The system control 122 includes a set of modules connected together by a backplane. These include a CPU module 119 and a pulse generator module 121 which connects to the operator console 100 through a serial link 125. It is through this link 125 that the system control 122 receives commands from the operator which indicate the scan sequence that is to be performed. The pulse generator module 121 operates the system components to carry out the desired scan sequence. It produces data which indicates the timing, strength and shape of the RF pulses which are to be produced, and the timing of and length of the data acquisition window. The pulse generator module 121 connects to a set of gradient amplifiers 127, to indicate the timing and shape of the gradient pulses to be produced during the scan. The pulse generator module 121 also receives patient data from a physiological acquisition controller 129 that receives signals from a number of different sensors connected to the patient, such as ECG signals from electrodes or respiratory signals from a bellows. And finally, the pulse generator module 121 connects to a scan room interface circuit 133 which receives signals from various sensors associated with the condition of the patient and the magnet system. It is also through the scan room interface circuit 133 that a patient positioning system 134 receives commands to move the patient to the desired position for the scan.

The gradient waveforms produced by the pulse generator module 121 are applied to a gradient amplifier system 127 comprised of $G_x$, $G_y$ and $G_z$ amplifiers. Each gradient amplifier excites a corresponding gradient coil in an assembly generally designated 139 to produce the magnetic field gradients used for position encoding acquired signals. The gradient coil assembly 139 forms part of a magnet assembly 141 which includes a polarizing magnet 140 and a whole-body RF coil 152. A transceiver module 150 in the system control 122 produces pulses which are amplified by an RF amplifier 151 and coupled to the RF coil 152 by a transmit/receive switch 154. The resulting signals radiated by the excited nuclei in the patient may be sensed by the same RF coil 152 and coupled through the transmit/receive switch 154 to a preamplifier 153. The amplified NMR signals are demodulated, filtered, and digitized in the receiver section of the transceiver 150. The transmit/receive switch 154 is controlled by a signal from the pulse generator module 121 to electrically connect the RF amplifier 151 to the coil 152 during the transmit mode and to connect the preamplifier 153 during the receive mode. The transmit/receive switch 154 also enables a separate RF coil (for example, a head coil or surface coil) to be used in either the transmit or receive mode.

The NMR signals picked up by the RF coil 152 are digitized by the transceiver module 150 and transferred to a memory module 160 in the system control 122. When the scan is completed and an entire array of data has been acquired in the memory module 160, an array processor 161 operates to Fourier transform the data into an array of image data. This image data is conveyed through the serial link 115 to the computer system 107 where it is stored in the disk memory 111. In response to commands received from the operator console 100, this image data may be archived on the tape drive 112, or it may be further processed by the image processor 106 and conveyed to the operator console 100 and presented on the display 104.

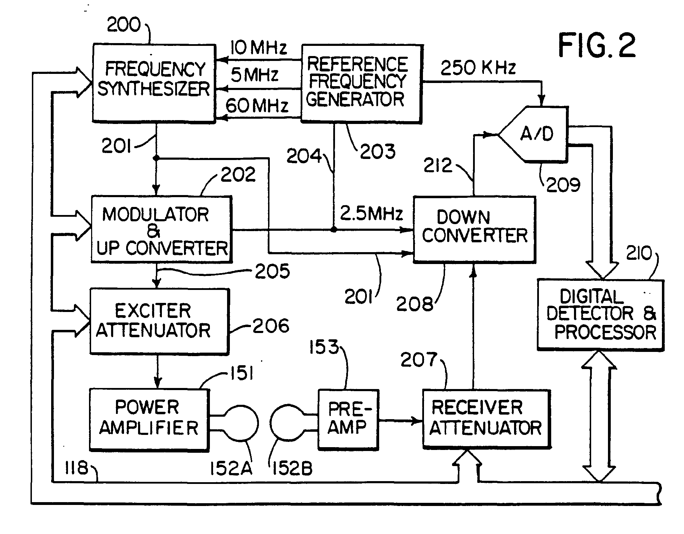

Referring particularly to Figs. 1 and 2, the transceiver 150 produces the RF excitation field B1 through power amplifier 151 at a coil 152A and receives the resulting signal induced in a coil 152B. As indicated above, the coils 152A and B may be separate as shown in Fig. 2, or they may be a single wholebody coil as shown in Fig. 1. The base, or carrier, frequency of the RF excitation field is produced under control of a frequency synthesizer 200 which receives a set of digital signals (CF) from the CPU module 119 and pulse generator module 121. These digital signals indicate the frequency and phase of the RF carrier signal produced at an output 201. The commanded RF carrier is applied to a modulator and up converter 202 where its amplitude is modulated in response to a signal R(t) also received from the pulse generator module 121. The signal R(t) defines the envelope of the RF excitation pulse to be produced and is produced in the module 121 by sequentially reading out a series of stored digital values. These stored digital values may, in turn, be changed from the operator console 100 to enable any desired RF pulse envelope to be produced.

The magnitude of the RF excitation pulse produced at output 205 is attenuated by an exciter attenuator circuit 206 which receives a digital command, TA, from the backplane 118. The attenuated RF excitation pulses are applied to the power amplifier 151 that drives the RF coil 152A. For a more detailed description of this portion of the transceiver 122, reference is made to U. S. Patent No. 4,952,877.

Referring still to Fig. 1 and 2 the signal produced by the subject is picked up by the receiver coil 152B and applied through the preamplifier 153 to the input of a receiver attenuator 207. The receiver attenuator 207 further amplifies the signal by an amount determined by a digital attenuation signal (RA) received from the backplane 118.

The received signal is at or around the Larmor frequency, and this high frequency signal is down converted in a two step process by a down converter 208 which first mixes the NMR signal with the carrier signal on line 201 and then mixes the resulting difference signal with the 205 MHz reference signal on line 204. The down converted NMR signal is applied to the input of an analog-to-digital (A/D) converter 209 which samples and digitizes the analog signal and applies it to a digital detector and signal processor 210 which produces 16-bit in-phase (I) values and 16-bit quadrature (Q) values corresponding to the received signal. The resulting stream of digitized I and Q values of the received signal are output through backplane 118 to the memory module 160 where they are employed to reconstruct an image.

The 2.5 MHz reference signal as well as the 250 kHz sampling signal and the 5, 10 and 60 MHz reference signals are produced by a reference frequency generator 203 from a common 20 MHz master clock signal. For a more detailed description of the receiver, reference is made to U.S. Patent No. 4,992,736.

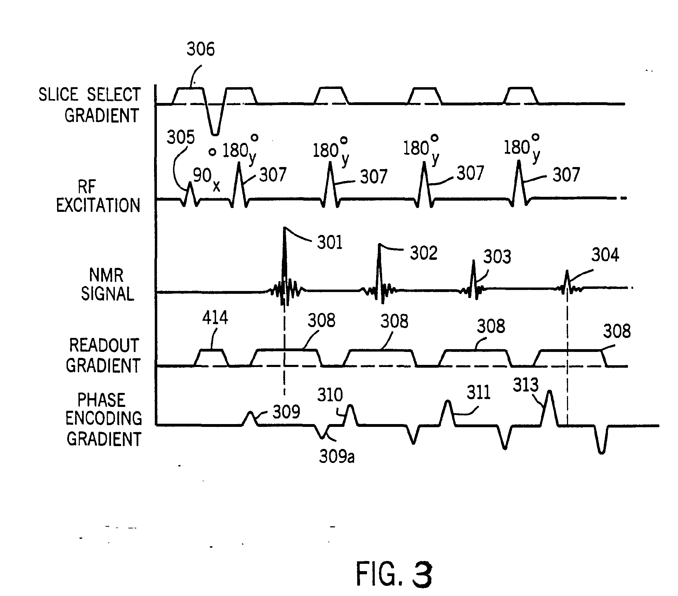

The MRI system of Fig. 1 performs a series of pulse sequences to collect sufficient NMR data to reconstruct the desired image. Referring particularly to Fig. 3, the fast spin echo MR pulse sequence employed to practice the preferred embodiment of the invention for $T_2$-weighted imaging is a 2DFT RARE sequence in which a plurality of MR echo signals are acquired. For clarity, only four echo signals 301-304 are shown in Fig. 2, but it can be appreciated that more may be produced and acquired. These MR echo signals are produced by a 90 degree RF excitation pulse 305 which is generated in the presence of a $G_z$ slice select gradient pulse 306 to provide transverse magnetization in a slice through the patient. This transverse magnetization is refocused by selective refocusing pulses 307 (which may have 180° flip angle) to produce the MR spin echo signals 301-304 that are acquired in the presence of $G_x$ readout gradient pulses 308. Each MR spin echo signal 301-304 is separately phase encoded and subsequently rewound by respective $G_y$ phase encoding pulses 309-313. The amplitude of the phase encoding pulse varies and it is stepped through discrete values to acquire separate "views", or samples of $k_y$ space. Each MR spin echo signal is acquired by digitizing samples of each signal, and as a result, at the completion of a scan for one image, a two-dimensional array of "k-space" data are acquired from which an image may be produced by performing a 2D Fourier transformation as described above.

Since the amplitude of the MR spin echo signals 301-304 decrease as a function of the $T_2$ decay constant of the spins being imaged, a number of measures are taken to reduce the spacing between the echo signals. These measures include the application of RF echo pulses 307 with minimal duration, and the use of high bandwidth and/or lower resolution readouts. Both result in less time to play out the associated gradient waveforms with a consequent reduction in the spacing between the MR signals 301-304. The RF refocusing pulses 307 are designed using the methods disclosed in U.S. patent nos. 5,315,249 and 5,345,176 and their duration is reduced, for example, from 3.2 ms to 1.92 ms. The duration of the 90 degree RF excitation pulse 305 is reduced from 4 ms to 2.4 ms. The duration of the readout of each MR echo signal is reduced by using in-plane zero padding in the reconstruction process. This is a well-known technique which enables fewer k-space samples to be acquired along the x or y axes. The missing samples are filled in with zeroes. In the preferred embodiment this enables fewer than 256 samples to be acquired during the readout of each MR signal 301-304 with a consequent reduction in echo spacing.

A second preferred embodiment of the invention employs a fast gradient-recalled echo pulse sequence to acquire the prescribed number of image slices. This pulse sequence is preferred when $T_1$-weighted abdominal images are to be acquired. The preferred pulse sequence is described in U.S. Pat. No. 5,291,891.

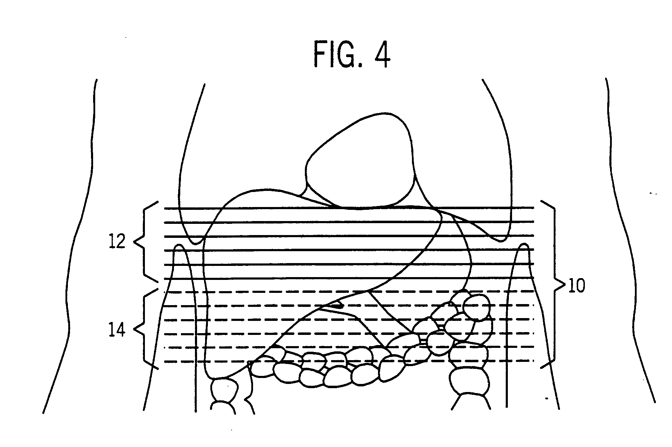

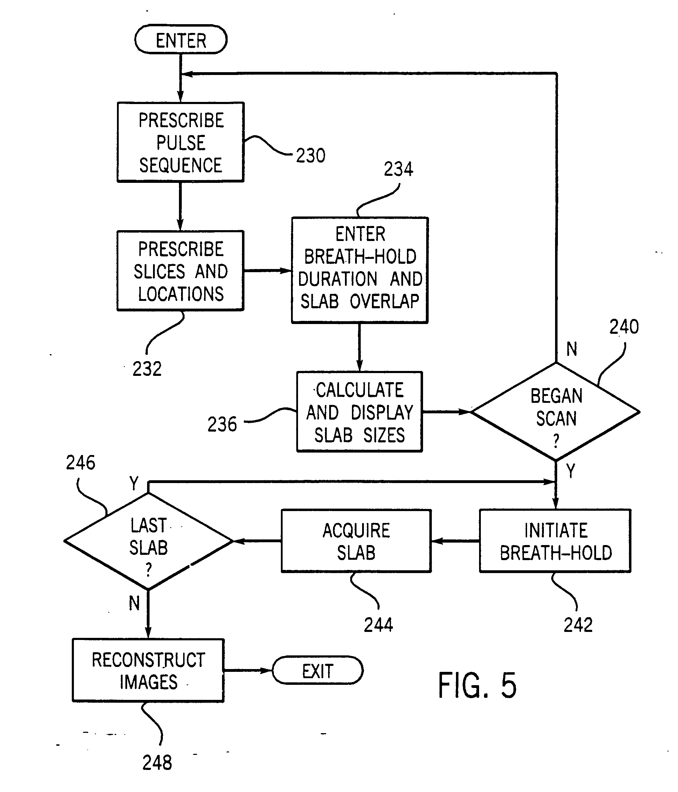

The preferred embodiment of the invention employs a 2D imaging sequence such as the FSE pulse sequence of Fig. 3 under the direction of a program executed by the MRI system of Fig. 1. The steps performed by this program are shown in the flow chart of Fig. 5 which will be explained using an example abdominal scan of the liver illustrated in Fig. 4.

Referring particularly to Fig. 4, the scan is performed by acquiring MRI data for twelve two dimensional images, oriented as twelve axial slices 10. However, rather than using the usual acquisition order in which data is acquired in an interleaved manner from all twelve slices, the slices are grouped into slabs. In the example shown, two slabs 12 and 14 are defined and each slab 12 and 14 has six slices. The main criteria for defining the number of slices in each slab is that the entire slab can be acquired using the chosen pulse sequence in a single patient breath-hold. The scan is then comprised of acquiring MRI data from each of a plurality of slabs during each of a corresponding succession of breath-holds. In the example of Fig. 4, two breath-holds are required to complete the scan.

Referring particularly to Fig. 5, when the procedure is started the operator is prompted to input the prescription for the pulse sequence that is to be performed as indicated at process block 230. This prescription includes such parameters as the number of echoes to be acquired in each shot if the above-described FSE pulse sequence is used. Other fast pulse sequences such as a spoiled gradient recalled echo sequence for $T_1$-weighted images may also be prescribed.

The number and location of the slices to be acquired during the scan are than prescribed as indicated at process block 232. These parameters will, of course, depend on the abdominal anatomy being imaged, the desired resolution and the field of view. In a scan of the liver using the above described FSE pulse sequence, for example, anywhere from 8 to 12 axial slices might be prescribed.

As indicated at process block 234 the operator then enters a breath-hold duration number which indicates the time interval available to acquire each slab of data. This time period may be preset to a "normal" value such as 25 seconds, and this may be increased or decreased depending on the condition and capability of the patient. The breath-hold duration is automatically reduced to the minimum possible duration as required by the user-specified repetition time (TR) and phase encoding resolution. In addition, a slab overlap number is entered. This number indicates the amount adjacent slabs are to overlap to ensure that all of the anatomy is acquired if the patient does not perform a breath-hold at the identical position for each slab acquisition.

As indicated at process block 236, the system then calculates the number of separate slabs to be acquired and the number of slices in each slab. This calculation is based on the previously entered data with the objective of acquiring as many prescribed slices in each breath-hold as possible. For example, if the calculations indicate that six slices can be acquired in the specified breath-hold duration, then each slab will contain up to six slices, and the minimum number of slabs/breath-holds needed in the scan is equal to the total number of prescribed slices divided by six. This information is displayed to the operator, and if satisfactory as indicated at decision block 240, the scan is begun. Otherwise, the system loops back to enable the operator to change the prescribed scan. For example, if the patient is reasonably healthy and able to hold their breath easily, the breath-hold timing can be increased.

When the scan is begun the patient is signaled to start a breath-hold as indicated at process block 242. When a breath-hold is established the acquisition of one slab of MR image data is acquired as indicated at process block 244. The detection of breath-hold initiation can also be done automatically using a system such as that described in U.S. Patent No. 5,363,844 entitled *"Breath-Hold Monitor for MR Imaging"*.

The acquisition of a slab of MR imaging data is performed using the prescribed pulse sequence. Slices within the slab are acquired in the well-known interleaved manner such that pulse sequences can be performed without delay and without saturating spins in adjacent slices. After the slab is acquired, the patient is instructed, or signaled to breathe. If the last slab has been acquired as determined at decision block 246, the slice images are reconstructed as indicated at process block 248, otherwise, the system loops back to acquire the next slab.

In an alternative embodiment of the invention a navigator pulse sequence is used during the data acquisition phase of the process to locate the position of the liver/diaphragm boundary during each breath-hold. Location of the top of the liver at the beginning of each breath-hold allows correction of slice locations in subsequent breath-holds relative to the first slab of slices. This liver/diaphragm position information can be used retrospectively to automatically register the slabs with each other as discussed above, or preferably, this position information can be used prospectively to adjust the scan parameters prior to slab acquisition as will be described in more detail below.

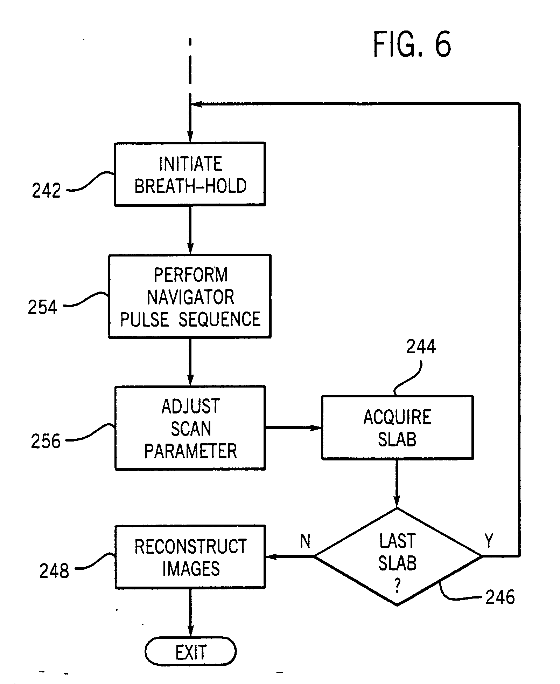

Referring particularly to Fig. 6 the alternative preferred embodiment is substantially the same process as described above except for the data acquisition steps. After each breath-hold is initiated at process block 242 a navigator pulse sequence is performed at process block 254 to acquire an NMR navigator signal. This navigator pulse sequence is a measurement in which a column of spins is excited by a two-dimensional RF excitation pulse. This column of spins is positioned by the operator and is typically located at the right side of the abdomen, and transecting the diaphragm near the dome of the liver. A subsequent NMR signal is acquired in the presence of a readout gradient ($G_z$ in the preferred embodiment) directed along the lengthwise dimension of the excited column, and the 256 samples of the NMR navigator signal are Fourier transformed by the array processor 161. The two-dimensional excitation RF pulse is a 3000 mm diameter Gaussian excitation of 12 msec. duration which produces a 90° flip angle. As described, for example, in U.S. Patent No. 4,812,760, such two-dimensional RF pulses are produced in the presence of two gradient fields ($G_x$ and $G_y$ in the preferred embodiment). The receiver low pass filter is set for a 260 mm field of view along the excited column (z axis). The NMR signal is sampled at 256 points during a 4 msec. sample period and a pulse repetition rate of TR = 70 msec. is employed. The liver/diaphragm position in the excited column appears as an inflection in the acquired and Fourier transformed NMR navigator signal. The position of this inflection is determined using an edge detection (derivative filter) technique which includes a 20-sample wide averaging filter to reduce higher frequency noise, followed by a 5-sample high pass filter which extracts the liver/diaphragm edge.

Other navigator signal acquisitions may also be employed. For example, a 2D sagital or coronal slice may be acquired,and analyzed to measure respiration motion.

As indicated in Fig. 6 by process block 256, the liver/diaphragm position information is used to alter a system scan parameter. During the acquisition of the first slab the liver/diaphragm position is stored as a patient reference position and the first slab of slices is acquired without scan parameter adjustment. During the acquisition of subsequent slabs, however, the measured liver/diaphragm position is compared with the stored patient reference position, and the difference is used to adjust a scan parameter. In the preferred embodiment the frequency of the RF excitation pulses are changed by an amount which shifts the location of the acquired slices along the axial direction by an amount equal to the calculated difference:

$$\Delta f = \gamma G_z \Delta z$$

where:

$\gamma$ = the gyromagnetic ratio for the excited spins,

$G_z$ = the gradient field strength along the z axis,

$\Delta z$ = difference in position of the liver/diaphragm position from the reference between breath holds.

This adjustment is made by changing the frequency of the RF carrier signal produced at output 201 (Fig. 2) as described above. The subsequently acquired slab is thus shifted to follow the motion of the patient and acquire slices which are registered with those in the first, reference, slab. When the scan is completed, the slice images are reconstructed at process block 248 to provide the prescribed set of slice images without the need for a retroactive registration step.

For completeness, various aspects of the invention are set out in the following numbered clauses:-

    1, A method for producing a set of images with a magnetic resonance imaging (MRI) system, the steps comprising:

        a) prescribing a pulse sequence (230);

        b) prescribing the number and location of the set of images (232);

        c) grouping the set of images into a plurality of slabs (236), each slab (12, 14) containing a plurality of adjacent images;

        d) acquiring the set of images by iteratively acquiring each slab (12, 14) by:

            i) initiating a patient breath-hold (242);

            ii) performing the prescribed pulse sequence with the MRI system to acquire MR image data (244) from the patient for each of the prescribed images in the slab; and

e) reconstructing the set of images (248) using the MR image data acquired in step d).

2. The method as recited in clause 1 in which step e) includes registering the acquired slabs (12, 14).

3. The method as recited in clause 1 in which the set of images are two-dimensional images taken through a corresponding set of adjacent parallel slices (10) through the patient.

4. The method as recited in clause 3 in which the set of images are axial images.

5. The method as recited in clause 3 in which the set of images are located in the abdomen of the patient.

6. The method as recited in clause 1 in which the pulse sequence is a fast spin echo pulse sequence (Fig 3).

7. The method as recited in clause 1 in which the MR image data is acquired for the images in each slab in step d) ii) in an interleaved order.

8. The method as recited in clause1 in which the grouping of the set of images into a plurality of slabs is performed by calculating the number of prescribed images that can be acquired with the prescribed pulse sequence in a patient breath-hold.

9. The method as recited in clause 8 which includes manually entering information (234) indicative of the patient's breath-hold duration.

10. The method as recited in clause 1 in which the grouping of the set of images into a plurality of slabs includes entering information (234) indicative of the amount of overlap of adjacent acquired slabs (12, 14).

11. The method as recited in clause 1 in which step d) includes:

performing a navigator pulse sequence (254) with the MRI system after the initiation of a patient breath-hold to acquire navigator data; and in which step e) includes registering the acquired slabs (12, 14) using the acquired navigator data.

12. The method as recited in clause 1 in which step d) includes:

performing a navigator pulse sequence (254) with the MRI system after the initiation of a patient breath-hold to acquire navigator data; and

adjusting a scan parameter on the MRI system (256) in response to the acquired navigator data to adjust the location of the slab of images subsequently acquired in step d) ii).

13. The method as recited in clause 12 in which the scan parameter is the frequency of an RF excitation pulse (305, 307) produced during the performance of the prescribed pulse sequence.

14. The method as recited in clause 13 in which the scan parameter is adjusted by changing the frequency of an RF carrier signal (201) produced by a transceiver (150) in the MRI system.

15. A method for producing a set of images with a magnetic resonance imaging (MRI) system, the steps comprising:

a) grouping the set of images into a plurality of slabs (236), each slab (12, 14) containing a plurality of adjacent images;

b) initiating a patient breath-hold (242);

c) performing a navigator pulse sequence (254) with the MRI system to acquire navigator data indicative of patient position;

d) adjusting a scan parameter on the MRI system (256) in response to the navigator data;

e) performing a pulse sequence on the MRI system with the adjusted scan parameter to acquire MR image data (244) from the patient for each of the images in one slab;

f) repeating steps b), c), d) and e) until MR image data has been acquired from all of the slabs (12, 14); and

g) reconstructing the set of images (248) from the acquired MR image data.

16. The method as recited in clause 15 in which the scan parameter is the frequency of an RF excitation pulse (305, 307) in the pulse sequence.

17. The method as recited in clause 15 in which the pulse sequence is a fast spin echo pulse sequence (Fig 3).

18. The method as recited in clause 15 in which the navigator data is indicative of the position of the patient's diaphragm.

**Claims**

1. A method for producing a set of images with a magnetic resonance imaging (MRI) system, the steps comprising:

   f) prescribing a pulse sequence (230);

   g) prescribing the number and location of the set of images (232);

   h) grouping the set of images into a plurality of slabs (236), each slab (12, 14) containing a plurality of adjacent images;

   i) acquiring the set of images by iteratively acquiring each slab (12, 14) by:

   i) initiating a patient breath-hold (242);

   ii) performing the prescribed pulse sequence with the MRI system to acquire MR image data (244) from the patient for each of the prescribed images in the slab; and

   j) reconstructing the set of images (248) using the MR image data acquired in step d).

2. The method as recited in claim 1 in which step e) includes registering the acquired slabs (12, 14).

3. The method as recited in claim 1 in which the set of images are two-dimensional images taken through a corresponding set of adjacent parallel slices (10) through the patient.

4. The method as recited in claim 1 in which the MR image data is acquired for the images in each slab in step d) ii) in an interleaved order.

5. The method as recited in claim1 in which the grouping of the set of images into a plurality of slabs is performed by calculating the number of prescribed images that can be acquired with the prescribed pulse sequence in a patient breath-hold.

6. The method as recited in claim 1 in which the grouping of the set of images into a plurality of slabs includes entering information (234) indicative of the amount of overlap of adjacent acquired slabs (12, 14).

7. The method as recited in claim 1 in which step d) includes:

performing a navigator pulse sequence (254) with the MRI system after the initiation of a patient breath-hold to acquire navigator data;

and in which step e) includes registering the acquired slabs (12, 14) using the acquired navigator data.

8. The method as recited in claim 1 in which step d) includes:

   performing a navigator pulse sequence (254) with the MRI system after the initiation of a patient breath-hold to acquire navigator data; and

   adjusting a scan parameter on the MRI system (256) in response to the acquired navigator data to adjust the location of the slab of images subsequently acquired in step d) ii).

9. A method for producing a set of images with a magnetic resonance imaging (MRI) system, the steps comprising:

   h) grouping the set of images into a plurality of slabs (236), each slab (12, 14) containing a plurality of adjacent images;

   i) initiating a patient breath-hold (242);

   j) performing a navigator pulse sequence (254) with the MRI system to acquire navigator data indicative of patient position;

   k) adjusting a scan parameter on the MRI system (256) in response to the navigator data;

   l) performing a pulse sequence on the MRI system with the adjusted scan parameter to acquire MR image data (244) from the patient for each of the images in one slab;

   m) repeating steps b), c), d) and e) until MR image data has been acquired from all of the slabs (12, 14); and

   n) reconstructing the set of images (248) from the acquired MR image data.

10. The method as recited in claim 9 in which the scan parameter is the frequency of an RF excitation pulse (305, 307) in the pulse sequence.

FIG.1

FIG.2

FIG. 3

FIG. 4

FIG. 5

FIG. 6

EXIT